# FASCICULE DE BREVET EUROPEEN

(11) **EP 2 056 843 B1**
(45) Date de publication et mention de la délivrance du brevet: **16.10.2013**
(21) Numéro de dépôt: 07803123.4
(22) Date de dépôt: 31.08.2007
(51) Int. Cl.: A61K 31/7004, A61K 36/54, A61P 31/10, A61P 31/00, A61P 17/10, A61P 17/08, A61P 17/00

(54) **UTILISATION DE SUCRES EN C7 DANS LA PRÉVENTION ET LE TRAITEMENT DES MYCOSES**
VERWENDUNG VON C7-ZUCKERN ZUR PRÄVENTION UND BEHANDLUNG VON MYKOSEN
USE OF C7 SUGARS IN PREVENTION AND TREATMENT OF MYCOSES

(30) Priorité: 31.08.2006 FR 0607651
(43) Date de publication de la demande: 13.05.2009
(73) Titulaire: Laboratoires Expanscience, 92400 Courbevoie (FR)
(72) Inventeur: MSIKA, Philippe, 78000 Versailles (FR)
(74) Mandataire: Regimbeau
(86) Numéro de dépôt international: PCT/EP2007/059136
(87) Numéro de publication internationale: WO 2008/025847

(56) Documents cités:
- WO-A-01/68040
- DE-A1- 19 852 508
- FR-A- 2 869 541
- FR-A1- 2 869 543
- SHAW P E ET AL: "HIGH PERFORMANCE LIQUID CHROMATOGRAPHIC ANALYSIS OF D MANNO HEPTULOSE PERSEITOL GLUCOSE AND FRUCTOSE IN AVOCADO CULTIVARS" JOURNAL OF AGRICULTURAL AND FOOD CHEMISTRY, AMERICAN CHEMICAL SOCIETY. WASHINGTON, US, vol. 28, no. 2, 1980, pages 379-382, XP009053733 ISSN: 0021-8561
- BREDIF S ET AL: "Avocado sugars are effective inducers of cutaneous defensive functions" JOURNAL OF INVESTIGATIVE DERMATOLOGY, vol. 126, no. Suppl. 3, août 2006 (2006-08), page 52, XP009081682 & 36TH ANNUAL MEETING OF THE EUROPEAN-SOCIETY-OF-DERMATOLOGY-RESEARCH (ESDR); PARIS, FRANCE; SEPTEMBER 06 -07, 2006 ISSN: 0022-202X
- "Avocado sugars are effective inducer of cutaneous defensive functions" JOURNAL OF THE AMERICAN ACADEMY OF DERMATOLOGY, C.V. MOSBY, ST. LOUIS, MO, US, vol. 56, no. 2, 20 janvier 2007 (2007-01-20), page AB84, XP005833192 ISSN: 0190-9622
- JOLY S ET AL: "Human [beta]-Defensins 2 and 3 Demonstrate Strain-Selective Activity against Oral Microorganisms", JOURNAL OF CLINICAL MICROBIOLOGY 200403 US LNKD- DOI:10.1128/JCM.42.3.1024-1029.2004, vol. 42, no. 3, March 2004 (2004-03), pages 1024-1029, ISSN: 0095-1137
- DATABASE EMBASE [en ligne] ELSEVIER SCIENCE PUBLISHERS, AMSTERDAM, NL Mai 2006 KWON K -S ET AL: 'Immunohistochemical study of human [beta] defensin-2 expression in superficial mycosis' Database accession no. EMB-2006437114 & KWON K -S ET AL: "Immunohistochemical study of human [beta] defensin-2 expression in superficial mycosis", KOREAN JOURNAL OF DERMATOLOGY 200605 KR, vol. 44, no. 5, May 2006 (2006-05), pages 538-544, ISSN: 0494-4739
- "120 Fungal skin infections" In: Beers, M., H. et al.: "The Merck Manual of diagnosis and therapy", 2006, Merck Research Laboratories, USA ISBN: 091 1910-18-2 pages 986-993, * pages 986-987 *

## Description

La présente invention concerne l'utilisation d'au moins un sucre en C7 choisi dans le groupe constitué par le mannoheptulose, les mélanges de mannoheptulose et de perséitol pour prévenir et/ou lutter contre la prolifération des levures présentes au niveau de la peau, des phanères et/ou muqueuses.

Classés dans le règne des *Fungi,* les champignons sont des organismes eucaryotes, hétérotrophes vis-à-vis du carbone, absorbotrophes, se reproduisant par sporulation. Les champignons microscopiques se subdivisent en moisissures, en dermatophytes et en levures (organismes unicellulaires). Certains champignons microscopiques peuvent provoquer chez l'homme des affections parasitaires de la peau, des phanères et des viscères.

Les mycoses sont des parasitoses causées par la prolifération de champignons microscopiques parasites de l'organisme. Parmi les mycoses les plus fréquentes, on peut citer les candidoses et les pityrosporoses, qui sont provoquées par la prolifération de levures de la peau.

Les candidoses sont des affections dues à une levure du genre candida. Il existe deux formes différentes de candidoses par leur évolution : les mycoses aiguës (les plus fréquentes), dues à des facteurs locaux, et les mycoses chroniques (rares), dont le traitement est plus difficile et qui sont parfois dues à un déficit du système immunitaire.

Les candidoses cutanées (touchant la peau) se localisent essentiellement au niveau des plis du corps et sont favorisées par la macération (excès d'humidité ou insuffisance d'assèchement de la peau). Les candidoses cutanées se traduisent par la présence d'un intertrigo, c'est-à-dire d'une lésion qui débute au fond des plis cutanés, qui devient érythémateuse (coloration rouge) et qui finit par suinter et entraîner un prurit (démangeaisons). Les candidoses cutanées peuvent également se rencontrer au niveau des ongles, à leur base. Elles provoquent ce qu'on appelle une tourniole, autrement dit un panaris superficiel.

Les candidoses buccales entraînent un muguet qui se caractérise par une coloration blanchâtre et rouge vif sur la face interne des joues. Les candidoses buccales apparaissent essentiellement chez les personnes souffrant d'une absence de salive (asialie). Chez les individus immunodéprimés (présentant une baisse des défenses immunitaires), comme ceux atteints par le VIH (sida) et chez les individus porteurs d'appareils dentaires, les candidoses buccales sont facilitées.

Les candidoses génitales sont à l'origine chez la femme de vulvo-vaginites avec pertes blanches et chez l'homme de balanite (inflammation du prépuce). Les candidoses génitales masculines sont à l'origine d'un prurit et d'un écoulement urétral assez fréquent. D'autres candidoses sont des aggravations de l'érythème fessier du nourrisson du fait de la macération et de l'augmentation du pH qui devient alcalin.

Les pityrosporoses sont des infections très fréquentes de la peau dues à une levure du genre *Pityrosporum.*

Il existe différents types de pityrosporoses, notamment :
- le pityriasis versicolor est une dermatose se caractérisant par l'apparition d'une rougeur diffuse (érythème) associée à une très légère desquamation (petite chute de pellicules de peau) et due à la germination d'une levure (du nom de *Pityriosporum orbiculare,* appelée également *Microsporun furfur* ou *Malassezia furfur*),
- le pityriasis du cuir chevelu se caractérise par la présence de pellicules (squames de taille variable) associées parfois à une démangeaison,
- la dermatose de Brocq se caractérise par la présence de tous petits médaillons de coloration rouge dont le centre est plus pâle, associée à une petite chute de lambeaux de peau, avec parfois des démangeaisons, et se localisant sur le devant de la poitrine,
- la dermite séborrhéique se caractérise par la présence de petites plaques de coloration rougeâtre ou parfois jaune-orangée recouvertes de squames (petits lambeaux de peau) d'allure liquidienne (gras), sans démangeaisons. Ces lésions dermatologiques se rencontrent sur le haut du visage à la lisière du cuir chevelu, des sourcils et au niveau des ailes du nez
- la folliculite pityriosporique se localise au niveau du dos. Elle se caractérise par de petits boutons en saillie et s'accompagne d'une légère démangeaison.

Des traitements pour prévenir ou traiter ces mycoses, ou leurs manifestations, sont connus mais l'homme du métier est toujours à la recherche de traitements alternatifs.

Or, les inventeurs ont découvert, de manière inattendue et surprenante, que des sucres en C7, le mannoheptulose et les mélanges de le mannoheptulose et de perséitol, en particulier ceux extraits de l'avocat, permettaient de provoquer l'agglomération ou l'agrégation physique de ces champignons les empêchant de pénétrer dans les cellules et par voie de conséquence d'inhiber leur pathogénicité.

Le D-mannoheptulose, premier cétoheptose identifié en 1916 par La Forge, de formule générale (I) 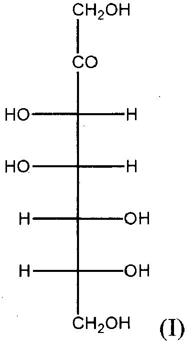 se retrouve dans certaines plantes, en particulier dans la luzerne (*Medicago sativa L.*), dans l'avocat, dans la figue (*Ficus officinalis* L.) dans l'orpin (*Sedum spectabile Bor.*) et dans la primevère (*Primula officinalis* Jacq.). Toutefois, c'est dans l'avocat, que l'on retrouve les teneurs les plus importantes en D-mannoheptulose. Le D-mannoheptulose a déjà été utilisé dans des applications thérapeutiques. Par exemple, la demande de brevet WO95/03809 décrit l'utilisation du D-mannoheptulose, en tant qu'inhibiteur de glucokinase, pour inhiber le développement des cellules tumorales et la demande US2003/0092669 décrit un complément alimentaire oral comprenant du D-mannoheptulose, qui permet de diminuer le taux d'insuline et qui permet ainsi une perte de poids.

Le perséitol, forme polyol du D-mannoheptulose, de formule générale (II) 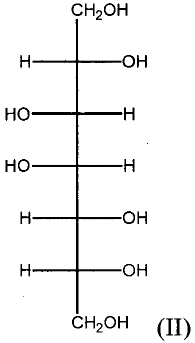 se retrouve également dans l'avocat, en particulier dans le fruit ou dans le noyau de l'avocat.

D'après la publication «Search for pharmacochemical leads from tropical rainforest plants », Hitotaka Shibuya et al. Pure Appl. Chem., vol. 71, n°6, pp 1109-1113, 1999, le perséitol, associé à un ion potassium, permet d'inhiber l'incorporation de leucine-3H dans des cellules tumorales d'ascite sarcomateuse d'Ehrlich.

L'utilisation de ces sucres (perséitol et D-mannoheptulose) pour stimuler la synthèse des beta-défensines humaines (en particulier HBD-2) a déjà été décrite (WO 2005/115421).

L'utilisation de ces sucres dans la prévention et le traitement de maladies liées à une déficience du système immunitaire a été décrite (FR2869543).

L'invention a pour objet l'utilisation d'au moins un sucre en C₇ choisi dans le groupe constitué par le mannoheptulose, les mélanges de mannoheptulose et de perséitol, pour la fabrication d'une composition destinée à la prévention et/ou au traitement, chez le bébé et l'enfant de bas âge, de mycoses choisies dans le groupe constitué par les candidoses, et les pityrosporoses choisies dans le groupe constitué par le pityriasis versicolor, la folliculite pityrosporique et la dermite séborrhéique.

Les inventeurs ont constaté que lorsque ces sucres en C7 sont mis en contact avec des levures particulières du genre candida et pityrosporum ces sucres sont capables de provoquer l'agrégation desdites levures. Une fois agrégées, lesdites levures ont une capacité restreinte à se mouvoir ; leur taille étant plus importante, ils ne peuvent plus pénétrer dans les kératinocytes.

Il est supposé que cette agrégation est due à la présence de résidus mannose à la surface de ces levures. En effet, alors que ces sucres en C7 sont capables de provoquer l'agglomération de ces levures, ils n'induisent pas l'agglomération de champignons dépourvus en surface de ces résidus ou de bactéries, telles que *S*. *aureus, P. aeruginosa* et *S. pyogenes* (qui n'ont pas de résidus mannose à leur surface).

Les levures visées dans l'invention présentent donc avantageusement des résidus mannose à leur surface.

En outre, les inventeurs ont découvert que ces sucres en C7 sont capables d'inhiber la pathogénicité des levures.

Un des modes d'action des levures consiste à moduler l'action proinflammatoire de l'hôte pour assurer leur survie (ainsi, la levure *M. furfur* est capable de diminuer la production de cytokines pro-inflammatoires censées lutter contre sa propagation telles que l'IL-1α, IL-6 et leTNF-α et d'augmenter la production de TGF β-1 (cytokine immunosuppressive et anti inflammatoire) par les kératinocytes). La suppression de ces interleukines permet aux levures de survivre dans l'hôte sans engendrer de sa part une réponse anti-inflammatoire.

Or, il a été constaté que lorsque les kératinocytes sont prétraités avec ces sucres en C7, ces kératinocytes retrouvent leur capacité à répondre avec une réaction proinflammatoire correcte vis-à-vis des levures. Les sucres en C7 sont donc capables d'inhiber la pathogénicité des levures et de rétablir les capacités de défenses des kératinocytes qui redeviennent intolérants aux levures.

Dans le cadre de la présente invention, les candidoses visées sont en particulier celles choisies dans le groupe constitué par les intertrigos candidosiques, les candidoses buccodigestives, les candidoses génitales adultes ou enfantines, les onyxis et périonyxis candidosiques, la candidose mucocutanée chronique et les folliculites candidosiques.

Les candidoses sont provoquées par des levures du genre *Candida,* et en premier lieu, *C. albicans.* Dans certaines conditions physiologiques (grossesse), pathologiques (diabète) ou iarrogènes (traitement par des hormones, en particulier des corticoïdes, par des antibiotiques ou des immunodépresseurs), les *Candida* peuvent devenir pathogènes et provoquer des manifestations cutanées et muqueuses diverses.

Outre les facteurs généraux cités précédemment, les intertrigos candidosiques sont favorisés par l'obésité, la macération et le manque d'hygiène.

Les candidoses buccodigestives peuvent atteindre tous les segments de l'appareil digestif. On distingue la perlèche, la chéilite, la stomatite (le muguet). Ces candidoses se marquent souvent par un érythème.

Les candidoses génitales sont surtout rencontrées chez les adultes ou par extension d'une dermite fessière chez l'enfant. On distingue la vulvovaginite candidosique, l'urétrite (fréquente chez les diabétiques), les balanites et balanoposthites et la candidose génitofessière infantile. La candidose génitofessière infantile atteint les enfants en bas âge et est généralement connue sous le nom d'érythème fessier (qui peut avoir différentes causes).

Les onyxis et périonyxis candidosiques sont favorisés par l'humidité.

Les candidoses mucocutanées chroniques sont des infections chroniques, cutanées et muqueuses, qui surviennent chez des patients atteints d'un déficit immunitaire.

Les folliculites candidosiques sont particulièrement associées à l'héroïnomanie et sont le reflet d'une septicémie.

Dans une variante préférée de l'invention, les candidoses sont choisies dans le groupe constitué par les candidoses génitofessières infantiles, en particulier l'érythème fessier.

Dans le cadre de la présente invention, les pityrosporoses visées sont celles choisies dans le groupe constitué par le pityriasis versicolor, la folliculite pityrosporique et la dermite séborrhéique.

Par définition, un état pelliculaire consiste en une desquamation non inflammatoire du cuir chevelu. La dermite séborrhéique s'en distingue par la présence d'un érythème et par une extension de la maladie en dehors du cuir chevelu. *Malassezia sp* y joue un rôle central, responsable d'une réaction inflammatoire conduisant aux altérations épidermiques.

Dans une variante préférée de l'invention, les pityrosporoses sont choisies dans le groupe constitué par la folliculite pityrosporique et la dermite séborrhéique.

Dans l'invention, les mycoses visées sont les candidoses (en particulier celles dues à la prolifération de *Candida spp,* en particulier de *C. albicans*) et les pityrosporoses (en particulier celles dues à la prolifération de *Malassezia spp,* en particulier de *M. fufur*).

Les levures du genre Malassezia (*Malassezia spp*.) sont associées à de nombreuses maladies systémiques et cutanées, soit qu'elles en sont la cause soit qu'elles y sont associées. Comme exemple de maladies liées à *Malassezia spp.,* on peut notamment citer le pityriasis versicolor, la dermite séborrhéique, les pellicules, la folliculite, la papillomatose confluente réticulée, et la blépharite séborrhéique. Il a également été montré que *Malassezia spp.* est associée à des infections plus profondes, telles que la mastite, la sinusite, l'arthrite purulente, l'otite maligne externe, la septicémie à champignon, l'angéite, la péritonite et la méningite. (Helen Ruth Ashbee, recent developments in the immunology and biology of Malassezia species, HEMS ImmunoL Med. Microbiol. 47 (2006) 14-23).

Or, les inventeurs ont montré que les sucres en C7 selon l'invention sont capables de provoquer l'agglomération des levures (qui ont alors une taille trop importante et ne peuvent plus pénétrer dans les cellules) et de rééquilibrer la tolérance des kératinocytes vis-à-vis de ces levures. Ces levures perdent de leur capacité d'invasion et de leur pathogénicité et ceci sans que les sucres à ces concentrations n'aient de capacité à tuer ou inhiber la croissance des agresseurs (pas fongicidie ni de fongistase).

De plus, ces sucres en C7 ont un mode d'action qui est un mode physique et non chimique. Ainsi, ils présentent une efficacité à long terme, au contraire des effecteurs chimiques qui sont susceptibles d'induire des résistances de la part des pathogènes.

Cet effet à long terme est particulièrement intéressant car il permet de traiter les patients atteints de mycose insensibles aux traitements classiques existant à ce jour, en raison notamment d'une résistance aux effecteurs chimiques utilisés de la part des levures.

Les sucres en C7 selon l'invention sont donc utiles dans le traitement et la prévention de mycoses. Ils peuvent également être associés à d'autres médications pour le traitement de maladies (maladies cutanées principalement) où la prolifération des levures peut en être une manifestation sans en être la cause.

Selon une variante avantageuse de l'invention, les sucres en C7 selon l'invention sont utilisés dans le cadre d'un traitement de l'érythème fessier. Ils peuvent être utilisés seuls ou en association avec d'autres principes actifs.

Il est intéressant de noter que l'action physique des sucres en C7 selon l'invention sur l'agrégation des levures ne perturbe pas l'équilibre cutané, qui est particulièrement fragile et sensible chez les bébés et enfants en bas âge. Ainsi, ces sucres sont particulièrement adaptés pour le traitement des bébés et enfants de bas âge ou des personnes ayant un équilibre cutané fragile, pour qui les traitements classiques doivent être utilisés avec parcimonie en raison d'effets secondaires indésirés pouvant être sévères.

Les principes actifs pouvant être utilisés en association sont l'oxyde de zinc, le talc, l'oxyde de titane, la vitamine B5, les polyphénols, l'anti enzyme (uréase, lipase, protéase) de type isoflavone de soja, l'acétate d'oleyle, les emulsionnnant eau dans huile ou dans silicone qui protègent la peau de l'enfant.

Les principes actifs pouvant être utilisés en association sont également des oligomères procyanidoliques (OPC) et des anthocyanosides, tels que ceux décrits dans la demande EP 953 353.

Selon une autre variante avantageuse de l'invention, les sucres en C7 selon l'invention sont utilisés dans le cadre d'un traitement de la dermite séborrhéique. Ils peuvent être utilisés seuls ou en association avec d'autres principes actifs.

Les principes actifs pouvant être utilisés en association sont les antifongiques (kétoconazole, pyrithione zinc, pyroctone olamine, ...) classiques, les antilipases, les sels de sélénium et de lithium, les goudrons (coal tar, ichtyol pale, etc), les agents anti inflammatoires, les exfoliants ou kératoregulateurs (AHA, acides polyhydroxylés, BHA libres estérifiés ou substitués...).

Dans le cadre de l'invention, l'expression "sucre en C7" désigne des sucres dont le motif sucre comprend 7 atomes de carbone. Le sucre en C7 est choisi dans le groupe constitué par le mannoheptulose, et les mélanges de mannoheptulose et de perséitol.

La source de D-mannoheptulose et/ou de perséitol peut être un extrait hydrosoluble de sucres d'avocat ou d'une autre plante. Autrement, le D-mannoheptulose et le perséitol sont disponibles commercialement (origine synthétique). Selon une variante avantageuse de l'invention, la source de D-mannoheptulose et/ou de perséitol est un extrait hydrosoluble de sucres d'avocat.

L'extrait hydrosoluble de sucres d'avocat peut directement être obtenu à partir de n'importe quelle partie de l'avocat ou de l'avocatier, telle que le fruit, la peau, le noyau, la feuille ou les racines de l'avocatier. Il est aussi possible d'obtenir un extrait peptidique d'avocat à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile (extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. L'extrait est avantageusement obtenu à partir du fruit frais de l'avocatier. Les fruits pourront être choisis parmi les variétés *Hass, Fuerte, Ettinger, Bacon, Nabal, Anaheim, Lula, Real, Zutano, Queen, Criola Selva, Mexicana Cranta, Region Dschang, Hall, Booth, Peterson, Co*//*inson Redn,* plus avantageusement parmi les variétés *Hass, Fuerte et Reed.* De préférence, on retiendra les variétés *Hass, Fuerte, Ettinger et Bacon,* et plus avantageusement les variétés *Hass* et *Fuerte.*

Le fruit de l'avocatier est principalement constitué d'eau, de pulpe, d'huile et de noyau. Les proportions de ces constituants sont, à l'instar de toutes matières naturelles et végétales, extrêmement variables. Toutefois, on admet généralement les données de composition moyennes suivantes, exprimées en pourcentage de fruit frais, données dans le tableau 1 suivant :

**Tableau 1**

| | |
|---|---|
| Eau | 70-85 % |
| Protéines | 1,5-4,5 % |
| Lipides | 12-23 % |
| Sucres | 1,5-5 % |
| Fibres | 1,1-1,6 % |

De fait, l'avocat n'est pas particulièrement riche en polysaccharides. Cependant, la nature des monosaccharides solubles est tout à fait particulière, tels que le perséitol ou le D-mannoheptulose constitués de 7 atomes de carbone.

L'extrait hydrosoluble de sucres d'avocat est susceptible d'être obtenu par un procédé comprenant les étapes successives suivantes :
- obtention d'un tourteau d'avocat, avantageusement du fruit de l'avocat, par séchage de l'avocat puis extraction des lipides (huile) ; ensuite
- délipidation complète dudit tourteau, puis lavage à l'eau ou à un milieu hydroalcoolique puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ; et
- le cas échéant, concentration sous vide et conditionnement.

La première étape du procédé consiste à sécher le fruit puis à le déshuiler. Ainsi, après tranchage du fruit en fines lamelles, son séchage peut être réalisé par l'ensemble des techniques connues de l'homme de métier, parmi lesquelles on peut citer le séchage à l'air chaud, la lyophilisation ou encore le séchage osmotique. D'une façon générale, la température lors de cette étape de séchage sera avantageusement maintenue, quelle que soit la technique employée, inférieure ou égale à 80°C. Dans le cadre du présent procédé, pour des raisons de facilité de mise en oeuvre et pour des raisons de coût, le séchage en séchoirs ventilés, en couche mince et sous courant d'air chaud, à une température comprise entre 70 et 75°C est préféré. La durée de l'opération peut varier de 5 à 72 heures.

Les lipides du fruit séché sont par la suite extraits soit par voie mécanique dans une presse à vis continue, ou encore par voie chimique, à l'aide d'un solvant tel que l'hexane, dans un extracteur de type soxhlet ou dans un extracteur continu à bande, de type De Smet®, notamment selon le procédé décrit dans la demande FR 2 843 027, ou par un procédé utilisant le CO₂ supercritique. Parmi les intérêts majeurs du procédé, l'huile co-produite constitue un produit bien évidemment directement valorisable. C'est pourquoi, on préfère l'extraction des lipides par voie mécanique. Le fruit sec et déshuilé, encore appelé tourteau, peut subir ensuite les étapes suivantes :
- délipidation complète, notamment à l'acétone et/ou à l'éthanol,
- décantation puis lavage du tourteau à l'eau et/ou un mélange hydroalcoolique,
- centrifugation, filtration, récupération de la fraction soluble (élimination du gâteau),
- concentration,
- déminéralisation par échange d'ions
- ultrafiltration avec un seuil de coupure de 10 kDa,
- concentration sous vide, ajout de conservateur et conditionnement.

De façon générale, l'extrait aqueux final peut contenir en poids 0,1 à 20 % de matière sèche, avantageusement 1 à 10 % de matière sèche, encore plus avantageusement 3 à 5 % de matière sèche. La teneur en sucres en C7, c'est-à-dire en D-mannoheptulose et en perséitol, dans la matière sèche est avantageusement comprise entre 50 et 100 %, plus particulièrement entre 65 et 90% en poids, par rapport au poids total de la matière sèche. Les données analytiques moyennes d'une solution aqueuse à 5 % d'extrait sec, obtenue par le procédé décrit précédemment, sont données dans le tableau 2 suivant :

**Tableau 2**

| pH (dilution ¼) | | 3-5 |
|---|---|---|
| Absorbance (dilution ½) | 420 nm | Inférieure à 0,200 |
| | 550 nm | Inférieure à 0,050 |
| Sucres en C7 / matière sèche | | 50-100% |

La composition relative en sucres de l'extrait hydrosoluble, en poids par rapport au poids total de la matière sèche de l'extrait, répond avantageusement aux critères suivants (composition relative déterminée par HPLC ; high performance liquid chromatography = chromatographie liquide haute performance):
- D-mannoheptulose 0 à 100%, en particulier 5 à 80 %,
- Perséitol 0 à 100%, en particulier 5 à 80 %,
- Saccharose inférieur à 10%,
- Glucose inférieur à 10%,
- Fructose inférieur à 10%.

L'extrait hydrosoluble de sucre d'avocat comprend avantageusement, par rapport au poids total de la matière sèche, 10 à 80% en poids de D-mannoheptulose plus avantageusement 15 à 70 % en poids de D-mannoheptulose. L'extrait hydrosoluble de sucre d'avocat comprend avantageusement, par rapport au poids total de la matière sèche, 20 à 80% en poids de perséitol, plus avantageusement 25 à 70% en poids de perséitol.

De préférence, la composition relative en sucres de l'extrait hydrosoluble, en poids par rapport au poids total de la matière sèche de l'extrait, répond aux critères suivants (composition relative déterminée par HPLC) :
- D-mannoheptulose 25 à 60 %,
- Perséitol 25 à 60 %,
- Saccharose inférieur à 10 %,
- Glucose inférieur à 10 %,
- Fructose inférieur à 10 %.

D'une manière surprenante les inventeurs ont constaté un effet de synergie entre le D-mannoheptulose et/ou le perséitol et les sucres minoritaires (fructose, glucose, saccharose) présents dans l'extrait de sucres d'avocat.

L'extrait obtenu pourra éventuellement être lyophilisé dans le but d'obtenir une poudre solide (extrait sec), totalement hydrosoluble.

L'extrait obtenu pourra éventuellement être fractionné en chaque sucre purifié. Cette séparation et purification peut être réalisée par l'ensemble des techniques connues de l'homme de métier, parmi lesquelles on peut citer de façon non exhaustive la précipitation/filtration, la recristallisation, ou encore la séparation par chromatographie telle que le procédé I.S.M.B. (Improved Simulated Moving Bed).

Dans le cadre de la présente invention, la composition peut en outre comprendre (avec ou sans les composés listés précédemment) des acides organiques, des sucres métabolisables et non métabolisables par l'organisme, des excipients tels que des polyols, des maltodextrines, des dérivés de silice, des oxydes de titane, des glycols comme le pentylène glycol ou le butylène glycol, le caprilyl glycol, des actifs pour le maintien de la flore cutanée.

Dans le cadre de la présente invention, la composition peut en outre comprendre (avec ou sans les composés listés précédemment) au moins un composé choisi dans le groupe constitué par les glycollients, les actifs hydratants, les activateurs de la synthèse de kératine, les kératorégulateurs, les kératolytiques, les agents restructurant de la barrière cutanée (activateurs de la synthèse des lipides cutanés, agonistes PPARs ou Peroxysome Proliferator Activated Receptor), les activateurs de la différenciation des kératinocytes (rétinoïdes, calcidone®, le calcium, vitamine D3, vitamine C), les activateurs de la prolifération des kératinocytes (rétinoïdes et vitamine B5), les antibiotiques, les agents anti-bactériens, les composés antifongiques (sorbate de potassium, parabens, piroctone olamine, pyrithione zinc, cyclopyrox, sels de sélénium, et les régulateurs de la desquamation - coal tar et ichtyol pale -), les agents anti-viraux, les sébo-régulateurs, tels que les inhibiteurs de 5-alpha réductase, notamment l'actif 5-alpha Avocuta^{®} commercialisé par les Laboratoires Expanscience, les immunomodulateurs, tels que le tacrolimus, le pimécrolimus, les oxazolines, les conservateurs, les agents anti-irritants (eaux thermales et dérivés d'avoine), les agents apaisants, des filtres et écrans solaires, les agents anti-oxydants, les facteurs de croissance, les agents cicatrisants ou les molécules eutrophiques, les médicaments et les agents anti-inflammatoires, et les composés contenant des insaponifiables d'huiles végétales.

Les activateurs de la synthèse de kératine pouvant être utilisés dans le cadre de la présente invention en association avec les sucres en C7 selon l'invention sont avantageusement les rétinoïdes, les peptides de lupin (commercialisés par la société Silab), des protéines clés du stratum comeum ou granulosum (kératines).

Les antibiotiques pouvant être utilisés dans le cadre de la présente invention en association avec les sucres en C7 selon l'invention , sont avantageusement l'acide fucidique, la pénicilline, les tétracyclines, la pristinamycine, l'érythromycine, la clindamycine, la mupirocine, la minocycline, la doxycycline. Les agents anti-viraux pouvant être utilisés dans le cadre de la présente invention en association avec ces sucres en C7 sont avantageusement l'acyclovir et le valacyclovir. Les agents anti-irritants pouvant être utilisés dans le cadre de la présente invention en association avec ces sucres en C7 sont avantageusement la glycine, les sucres et/ou peptides de lupin, le Cyclocéramide^{®} (dérivé d'oxazoline).

Les agents apaisants pouvant être utilisés dans le cadre de la présente invention en association avec les sucres en C7 selon l'invention sont avantageusement l'alpha bisabolol, les dérivés de réglisse. Les kératorégulateurs pouvant être utilisés dans le cadre de la présente invention en association avec ces sucres en C7 sont avantageusement les alpha-hydroxy acides et leurs dérivés. Un kératolytique pouvant être utilisé dans le cadre de la présente invention en association avec ces sucres en C7, est notamment l'acide salicylique et ses dérivés. Les agents anti-oxydants pouvant être utilisés dans le cadre de la présente invention en association avec ces sucres en C7 sont avantageusement les vitamines (C, E), les oligo-éléments (cuivre, zinc, sélénium). Les facteurs de croissance pouvant être utilisés dans le cadre de la présente invention en association avec ces sucres en C7 sont avantageusement la becaplermine et le TGF-beta (Transforming Growth Factor bêta).

Les agents cicatrisants pouvant être utilisés dans le cadre de la présente invention en association avec les sucres en C7 selon l'invention sont avantageusement la vitamine A, le panthénol, l'Avocadofurane®, l'oxyde zinc, le magnésium, le silicium, l'acide madécassique ou asiatique.

Les médicaments pouvant être utilisés dans le cadre de la présente invention, en association avec les sucres en C7 selon l'invention, sont avantageusement les médicaments, appropriés pour une administration pour voie topique ou orale, pour la prévention et/ou le traitement de l'atopie (corticoïdes, émollients), de l'acné (antibiotiques, peroxyde de benzoyle, rétinoïdes, acide azélaïque, vitamine PP, zinc, cyclines), de l'eczéma (immunomodulateurs, émollients, huile de saumon, de bourrache, les pré biotiques, les pro biotiques) ou du psoriasis (corticoïdes, calcipotriol, calcitriol, tazarotène, huile de cade, acitrétine, PUVA thérapie). Les agents anti-inflammatoires pouvant être utilisés dans le cadre de la présente invention en association avec le D-mannoheptulose et/ou perséitol sont avantageusement des agents anti-inflammatoires stéroïdiens (AIS), tels que les corticoïdes, ou non-stéroïdiens (AINS).

Les agents restructurant de la barrière cutanée, permettant de stimuler la synthèse des lipides clés de l'épiderme, et pouvant être utilisés dans le cadre de la présente invention en association, avantageusement avec un effet de synergie, avec les sucres en C7 selon l'invention, sont avantageusement des concentrats de tournesol, encore plus avantageusement des concentrats de tournesol linoléiques, tels que l'actif commercialisé par les Laboratoires Expanscience, Soline^{®} (cf. la demande internationale WO 01/21150), des insaponifiables d'huile végétale, tel que l'Avocadofurane® (cf. la demande internationale WO 01/21150), des agonistes PPARs (rosiglitazone, pioglitazone). Les agents restructurant sont avantageusement présent en une proportion allant de 0,001 à 30 % en poids, par rapport au poids total de la composition ou du médicament. Les composés antifongiques pouvant être utilisés dans le cadre de la présente invention en association avec les sucres en C7, avantageusement le D-mannoheptulose et/ou le perséitol, sont avantageusement l'econazole et le ketoconazole.

Les conservateurs antiseptiques pouvant être utilisés dans le cadre de la présente invention en association avec les sucres en C7 selon l'invention , sont par exemple le triclosan, la chlorhéxidine, les ammoniums quaternaires. Les immnumodulateurs pouvant être utilisés dans le cadre de la présente invention en association, avantageusement avec un effet de synergie, avec les sucres en C7 selon l'invention sont avantageusement le tacrolimus, le pimécrolimus et les oxazolines.

Les oxazolines pouvant être utilisées dans le cadre de la présente invention en association, avantageusement avec un effet de synergie, avec les sucres en C7 selon l'invention sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide^{®}.

Les composés contenant des insaponifiables d'huiles végétales pouvant être utilisés dans le cadre de la présente invention en association, avantageusement avec un effet de synergie, avec les sucres en C7 selon l'invention sont avantageusement choisis dans le groupe constitué par les lipides furaniques d'avocat, les insaponifiables d'avocat et de soja, les concentrats d'huile de lupin, les concentrats d'huile de tournesol et leurs mélanges.

Les lipides furaniques d'avocat pouvant être utilisés dans le cadre de la présente invention en association, avantageusement avec un effet de synergie, avec les sucres en C7 selon l'invention sont avantageusement des 2-alkyl furanes naturels, notamment l'actif Avocadofurane^{®} commercialisé par les Laboratoires Expanscience, pouvant être obtenus par le procédé décrit dans la demande internationale WO 01/21605.

Les insaponifiables d'avocat et de soja pouvant être utilisés dans le cadre de la présente invention en association, avantageusement avec un effet de synergie, avec les sucres en C7 selon l'invention sont avantageusement un mélange d'insaponifiables d'avocat furanique et d'insaponifiables de soja, dans un rapport respectif d'environ 1/3-2/3. Les insaponifiables d'avocat et de soja sont encore plus avantageusement le produit Piasclédine^{®}, commercialisé par les Laboratoires Expanscience.

Les concentrats d'huile de lupin pouvant être utilisés dans le cadre de la présente invention en association, avantageusement avec un effet de synergie, avec les sucres en C7 selon l'invention sont avantageusement des concentrats obtenus par distillation moléculaire d'huile de lupin, avantageusement d'huile de lupin blanc doux, tels que ceux décrits dans la demande internationale WO 98/47479. Ils contiennent avantageusement environ 60% en poids d'insaponifiables.

Les concentrats d'huile de tournesol pouvant être utilisés dans le cadre de la présente invention en association, avantageusement avec un effet de synergie, avec les sucres en C7 selon l'invention sont avantageusement des concentrats de tournesol linoléiques, tels que l'actif commercialisé par les Laboratoires Expanscience, Soline^{®} (cf. la demande internationale WO 01/21150).

Selon une variante avantageuse de l'invention, la composition selon l'invention comprend en outre au moins un agent choisi dans le groupe constitué des antifongiques (classiques pharmaceutiques ou cosmétiques, tels que ceux définis précédemment), des antibactériens (tels que ceux définis précédemment), des antibiotiques (classiques ou endogènes, tels que ceux définis précédemment), les anti-viraux(tels que ceux définis précédemment), des extraits végétaux lipidiques contenant des insaponifiables tel que les concentrats de tournesol, les concentrats d'avocat et les concentrats de soja (tels que ceux définis précédemment), de l'oxyde de zinc, des vitamines (telles que celles définies précédemment), et des peptides et sucres végétaux (tels que ceux définis précédemment), les anti-inflammatoires (tels que ceux définis précédemment), les activateurs de la différenciation et/ou de la prolifération des kératinocytes (tels que ceux définis précédemment).

La composition (c'est-à-dire le produit final destiné à être commercialisé) comprend avantageusement 0,001 à 20% en poids, plus avantageusement 0,001 à 10% en poids, encore plus avantageusement 0,01 à 10% en poids, encore plus avantageusement 0,01 à 5% en poids, encore plus avantageusement 0,01 à 2% en poids de sucres en C7 selon l'invention, par rapport au poids total de la composition. Les sucres en C7 sont le mannoheptulose, et les mélanges de mannoheptulose et de perséitol. Selon une variante avantageuse de l'invention, la composition comprend avantageusement 0,01 à 20% en poids, plus avantageusement 0,01 à 10% en poids, encore plus avantageusement 0,05 à 5% en poids, encore plus avantageusement 0,05 à 2% en poids d'un extrait de sucres d'avocat tel que défini précédemment, par rapport au poids total de la composition.

Les modes d'administration, les posologies et les formes galéniques optimales de la composition peuvent être déterminés selon les critères généralement pris en compte dans l'établissement d'un traitement pharmaceutique ou dermatologique/ cosmétologique ou vétérinaire par voie locale ou systémique.

### Exemple 1 : préparation d'un extrait hydrosoluble de sucres d'avocat

50 kg d'avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80°C, sa durée est quant à elle de 48 heures. Une fois séchés, les fruits sont soumis à une pression à froid. Cette opération est réalisée sur une petite presse Komet® de laboratoire. 4 kgs de fruits secs délipidés dits de tourteau sont alors obtenus.

Les fruits délipidés sont alors broyés à froid puis extraits à reflux, en présence de 25 litres d'éthanol. La poudre épuisée en lipides est alors récupérée par filtration sur Büchner et séchée à l'étuve à 50°C, pendant 5 heures.

Le tourteau est alors lavé par un mélange hydroalcoolique 70/30 (10 litres) puis séparé par centrifugation. La fraction soluble (liquide) est reprise pour être purifiée et concentrée selon le mode opératoire suivant :
- *Déminéralisation à l'aide de résines ioniques :* déminéralisation des sucres en C7 par passage sur résines OH⁻, puis sur résine H⁺.
- *Ultrafiltration sur 10 000 Da :* l'ultrafiltration est réalisée avec un système équipé de 4 membranes de seuil de coupure 10 kDa.
- *Concentration sous vide :* la concentration de l'extrait purifié est réalisée à l'aide d'un évaporateur sous vide jusqu'à l'obtention d'une matière sèche voisine de 6 %.
- *Conditionnement :* la concentration de l'extrait est ajustée à 5 % de matière sèche, puis on réalise une filtration stérilisante avec une membrane de 0,2 µm de seuil de coupure.

Le tableau 3 suivant donne la composition de l'extrait de sucres d'avocat en C7, à 5 % de matière sèche, préparé suivant le procédé décrit ci-dessus :

**Tableau 3**

| **Aspect** | Solution de couleur jaune pâle |
|---|---|
| **Critères analytiques** | |
| Matière sèche | 5% |
| pH | 4,0 |
| Absorbance à 420 nm (dilution ½) | 0,008 |
| Absorbance à 550 nm (dilution ½) | 0,004 |

| **Composition (%/matière sèche)** | |
|---|---|
| Saccharose | 1,8 |
| Glucose | 3,0 |
| D-mannoheptulose | 38,6 |
| Fructose | 4,4 |
| Perséitol | 38,0 |

Suivant ce même procédé, on a préparé un autre extrait, dont la valeur du pH, l'absorbance et la teneur en sucres en C7 sont données dans le tableau 4 suivant. La teneur en sucres en C7 correspond à la somme du perséitol et du D-mannoheptulose analysée par HPLC.

**Tableau 4**

| Matière sèche | | 5 % |
|---|---|---|
| pH | | 4,0 |
| Absorbance (dilution ½) | 420 nm | 0,010 |
| | 550 nm | 0,005 |
| Sucres en C7 / matière sèche | | 83,6 |

### Exemple 2 Effets de l'extrait de sucres d'avocat sur la pathogénicité de levures

On a analysé les effets de l'extrait de sucres d'avocat décrit à l'exemple 1 (AV 119) sur les capacités d'invasion de levures dont *Malassezia furfur* (une levure dimorphe et lipo-dépendante qui est un commensale de la flore cutanée normale humaine) et *Candida albicans.* On a également analysé l'effet de ces levures et d'AV119 sur l'expression des cytokines proinflammatoires et immunomodulatrices des kératinocytes.

### 1. Micro-organismes utilisés

*Staphylococcus aureus* ATCC 29737, *Streptococcus pyogenes* ATCC 10389, *Pseudomonas aeruginosa* ATCC 25619, *Candida albicans* ATCC 53324 et *Malassezia furfur* ATCC 12078 sont obtenus auprès de l'American Type Culture Collection (Rockeville, MD USA).

Les souches bactériennes sont utilisées en tant que contrôle négatif et la levure C. *albicans* est utilisée en tant que contrôle positif. La cible principale de l'étude est *Malassezia furfur.*

### 2. Culture cellulaire et traitement

Les tests sont réalisés sur des kératinocytes humains normaux obtenus à partir d'échantillons chirurgicaux de peau normale d'adulte.

### 3. Etude de l'effet d'AV119 sur la viabilité et la croissance des micro-organismes :

Afin d'étudier les effets d'AV119 sur la viabilité de *S.aureus, P. aeruginosa, S. pyogenes, C. albicans* et *M. furfur,* on utilise différentes concentrations en AV119 (0,03 % à 0,5 % de matière sèche) pour déterminer s'il y a une inhibition de la croissance des bactéries et levures.

L'activité anti-microbienne est déterminée par la méthode modifiée de diffusion en gel d'agar de *Perez et al.* (Perez C. et al. An antibiotic assay by the agar well diffusion method. Acta Biologiae et Medicine Experimentalis. 1990 ; 15 : 113-115).

Les plaques sont incubées à 35 ± 2°C pendant 24 heures, sauf pour *C*. *albicans* (incubation à 30°C) et *M. furfur* (incubation à 30°C pendant 4 jours). La mesure du diamètre de la zone d'inhibition permet de conclure quant à l'effet anti-microbien.

La détermination des micro-organismes vivants a été faite après 24h de contact AV 119 / micro-organisme par comptage des unités formant colonie (UFC) sur le milieu agar sélectif afin d'exclure un effet anti-microbien direct d'AV119.

Les résultats obtenus, montrent que les micro-organismes analysés (*S. aureus P. aeruginosa, S. pyogenes, M. furfur* et *C*. *albicans)* sont tous résistants à AV119. En effet, à toutes les concentrations testées, aucun effet significatif sur la viabilité ou la croissance des bactéries et levures n'est observé.

Ces résultats permettent d'exclure un effet bactéricide direct d'AV119.

### 4. Essais d'invasion par S. aureus, P. aeruginosa et S. pyogenes

Les kératinocytes sont prétraités avec 0,1 % d'AV119 pendant 24 heures, puis infectés avec des suspensions de bactéries (à raison de 100 bactéries par cellule) pendant 2 heures à 37°C.

Les cellules sont ensuite lavées, soumises à une période d'incubation de 2 heures à 37°C puis lysées. Les lysats cellulaires sont alors dilués en PBS et déposés sur un milieu sélectif en agar afin de quantifier le nombre de bactéries intra-cellulaires vivantes.

AV119 ne modifie pas les capacités d'invasion de *S. aureus, P. aeruginosa et S. pyogenes.*

### 5. Essais d'invasion par M. furfur et C. albicans

Les kératinocytes humains sont prétraités pendant 24 heures avec 0, 1 % d'AV119 puis infectés par *M. furfur* (levure/ kératinocyte : 30/ 1) ou par *C*. *albicans* (levure / kératinocyte : 10/ 1) pendant 24, 48 ou 72 heures.

Ensuite, les cellules sont lavées, colorées (May-Grumwald-Giemsa) et observées au microscope.

Le pourcentage d'incorporation est évalué en comptant le nombre de kératinocytes dans lesquels *M. furfur* ou *C. albicans* a pénétré.

Les résultats montrent que les sucres inhibent l'absorption de *M. furfur* et *C. albicans (tableau 5).*

En effet, dans les kératinocytes traités avec AV119, on n'observe pas de pénétration des levures (pénétration observée dans les kératinocytes non traités).

**Tableau 5. Effet d'AV119 sur la capacité d'invasion de Malassezia furfur et Candida albicans**

| **Conditions de croissance** | **Nombre de levures internalisées** **(moyenne ± SD)** | | | |
|---|---|---|---|---|
| | ***M. furfur*** | | ***C. albicans*** | |
| | **48h** | **72h** | **48h** | **72h** |
| Keratinocyte-SFM medium | 155 ± 18 | 148 ± 16 | 225 ± 23 | 247 ± 31 |
| Keratinocyte-SFM medium + 0,025% AV119 | 150 ± 21 | 153 ± 15 | 195 ± 18 | 205 ± 27 |
| Keratinocyte-SFM medium + 0,050% AV119 | 140 ± 19 | 137 ± 13 | 203 ± 13 | 197 ± 31 |
| Keratinocyte-SFM medium + 0,1% AV119 | 14 ± 7 | 16 ± 4 | 36 ± 11 | 32 ± 5 |
| Keratinocyte-SFM medium + 0,2% AV119 | 15 ± 4 | 19 ± 3 | 41 ± 6 | 37 ± 3 |

### 6. Tests de floculation

La floculation est quantifiée en mesurant la densité optique (à 660 nm) d'une suspension de *M. furfur* et de *C. albicans* après agitation et une période de repos ultérieure afin de permettre aux floculats de sédimenter.

Les tests sont réalisés en présence de 0,1% d'AV119 ou de glucose (contrôle négatif) ou de mannane (contrôle positif).

Les tests de floculation sont réalisés en présence de différentes concentrations de CaCl₂, étant donné que les ions Ca²⁺ sont nécessaires à la floculation.

Les tubes sont agités pendant 15 minutes et laissés au repos pendant 15 min puis on mesure la densité optique à 660 nm.

On observe une agrégation ; les cellules de levures forment de larges agrégats.

**Tableau 6 Résultats (DO660) floculation de C. albicans**

| Log [Ca⁺⁺] | -1 | 0 | 1 | 2 |
|---|---|---|---|---|
| *C. albicans* | 1,95 | 1,97 | 1,82 | 1,91 |
| *C. albicans* + AV 119 | 1,93 | 1,2 | 0,7 | 0,6 |

**Table 7 Résultats (DO660) floculation de M. furfur**

| Log [Ca⁺⁺] | -1 | 0 | 1 | 2 |
|---|---|---|---|---|
| *M. furfur* | 2 | 2,1 | 1,98 | 1,99 |
| *M. furfur + AV119* | 1,98 | 1,1 | 0,5 | 0,5 |

On constate donc qu'en absence d'AV119, les levures *C. albicans* et *M. furfur* ne s'agrègent pas. L'ajout d'AV119 provoque la floculation des ces deux levures.

### 7. Analyse de l'expression de cytokines pro-inflammatoires par RT-PCR en temps réel

Pour déterminer si AV119 interfère avec le mécanisme d'action de *M. furfur,* l'expression de quelques cytokines, connues pour être régulées par la levure, est analysée.

L'expression des ARNm d'IL1α, IL6, IL8 et TGFβ1 dans les kératinocytes humains est déterminée par RT-PCR en temps réel.

Les cellules sont prétraitées avec 0,1 % d'AV 119 pendant 24 heures, puis traitées avec *M. furfur* (levure / kératinocytes : 30 / 1) pendant 24 et 48 heures.

Il est connu que *M. furfur* agit notamment en diminuant l'expression de la cytokine IL1α et en augmentant la production de TGFβ1. Ainsi, la levure module la réponse pro-inflammatoire de l'hôte pour permettre sa survie.

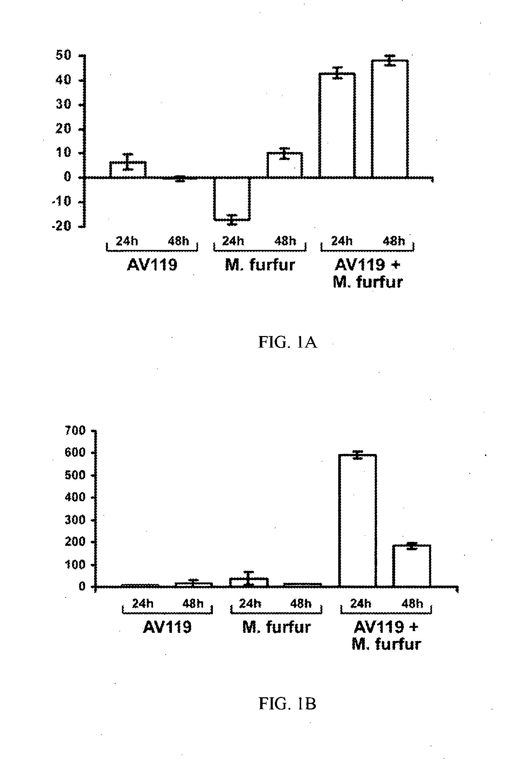

Les résultats (figures 1A à 1D) démontrent qu'AV119 permet aux kératinocytes de retrouver leur capacité à réagir à l'invasion par la levure avec une réaction proinflammatoire correcte :

### Légende des figures:

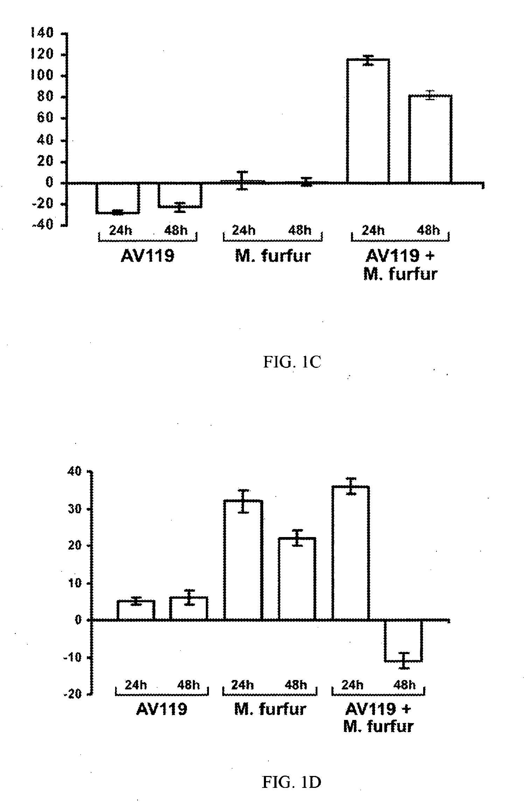

Figure 1A : augmentation du taux d'IL-1α (% par rapport au contrôle) pour, dans l'ordre de gauche à droite :
   AV119, 24 heures et 48 heures
   M. Furfur, 24 heures et 48 heures
   AV119 + M. furfur, 24 heures et 48 heures
Figure 1B : augmentation du taux d'IL-6 (% par rapport au contrôle) pour, dans l'ordre de gauche à droite :
   AV119, 24 heures et 48 heures
   M. Furfur, 24 heures et 48 heures
   AV119 + M. furfur, 24 heures et 48 heures
Figure 1C : augmentation du taux d'IL-8 (% par rapport au contrôle) pour, dans l'ordre de gauche à droite :
   AV119, 24 heures et 48 heures
   M. Furfur, 24 heures et 48 heures
   AV119 + M. furfur, 24 heures et 48 heures
Figure 1D : augmentation du taux de TGF-β (% par rapport au contrôle) pour, dans l'ordre de gauche à droite :
   AV119, 24 heures et 48 heures
   M. Furfur, 24 heures et 48 heures
   AV119 + M. furfur, 24 heures et 48 heures

Le traitement des kératinocytes (infectés par *M. furfur*) par AV119 entraîne une augmentation de la production des cytokines IL1α, IL6, IL8 par rapport aux kératinocytes non traités infectés par *M. furfur.* Cette augmentation atteint 43% pour Il-1α (figure 1A) après 24 heures de traitement, tandis que pour IL-6 et IL-8 elle atteint plus de 100% (figures 1B et 1C). A l'inverse, une diminution significative de la production de TGFβ1 est observée après 48 heures en réponse au traitement par AV 119 (figure 1D).

Ces résultats montrent que les kératinocytes retrouvent leur capacité de défense.

Finalement, l'éventuel relargage de métabolites de M. furfur dans les surnageants des kératinocytes traités a été recherché. Des kératinocytes traités ou non par AV119 sont incubés avec le surnageant obtenu de cellules traitées par AV119 et infectées par *M. furfur.* La réponse des kératinocytes n'est pas modifiée par la présence ou non du surnageant, indiquant l'absence d'un métabolite spécifique capable d'induire une réponse pro-inflammatoire dans les cellules traitées avec AV119 et infectées par M. *furfur.*

### N.B. : Analyse statistique :

Chaque expérience est répétée au moins 5 fois.

Les résultats sont exprimés sous la forme de la moyenne ± la déviation standard (SD).

Des tests ANOVA sont réalisés pour chaque expérience. La valeur de p est généralement évaluée entre 0,01 et 0,03, ce qui confirme la pertinence statistiques des résultats obtenus (p<0,05).

## Revendications

1. Utilisation d'au moins un sucre en C₇ choisi dans le groupe constitué par le mannoheptulose, les mélanges de mannoheptulose et de perséitol, pour la fabrication d'une composition destinée à la prévention et/ou au traitement, chez le bébé et l'enfant de bas âge, de mycoses choisies dans le groupe constitué par
- les candidoses, et
- les pityrosporoses choisies dans le groupe constitué par le pityriasis versicolor, la folliculite pityrosporique et la dermite séborrhéique, en particulier la dermite séborrhéique
en provoquant l'aggrégation des levures du genre candida et pityrosporum.

2. Utilisation selon la revendication 1, **caractérisée en ce que** les candidoses sont choisies dans le groupe constitué par les candidoses génitofessières infantiles, en particulier l'érythème fessier.

3. Utilisation selon l'une quelconque des revendications précédentes, **caractérisée en ce que** la source de sucres en C7 est un extrait hydrosoluble de sucres d'avocat.

4. Utilisation selon l'une quelconque des revendications précédentes, **caractérisée en ce que** la source de sucres en C7 est un extrait hydrosoluble de sucres d'avocat susceptible d'être obtenu par un procédé comprenant les étapes successives suivantes :
- obtention d'un tourteau d'avocat, avantageusement du fruit de l'avocat, par séchage de l'avocat puis extraction des lipides; ensuite
- cryobroyage, délipidation complète et extraction hydroalcoolique ou aqueuse dudit tourteau, puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ; et
- concentration sous vide et conditionnement.

5. Utilisation selon l'une quelconque des revendications précédentes, **caractérisée en ce que** la source de sucres en C7 est un extrait hydrosoluble de sucres d'avocat qui comprend en poids, par rapport au poids total de la matière sèche de l'extrait (composition relative déterminée par HPLC):
- D-mannoheptulose 1 à 90 %
- Perséitol 1 à 90 %
- Saccharose inférieur à 10%
- Glucose inférieur à 10%
- Fructose inférieur à 10%

6. Utilisation selon l'une quelconque des revendications précédentes, **caractérisée en ce que** la composition comprend 0,001 à 20% en poids de sucres en C7, par rapport au poids total de la composition.

7. Utilisation selon l'une quelconque des revendications 3 à 6, **caractérisée en ce que** la composition comprend 0,01 à 20% en poids dudit extrait hydrosoluble de sucres d'avocat, par rapport au poids total de la composition.

8. Utilisation selon l'une quelconque des revendications précédentes, **caractérisée en ce que** la composition comprend en outre au moins un actif choisi dans le groupe constitué par les antifongiques, les antibactériens, les antibiotiques, les anti-viraux, les extraits végétaux lipidiques contenant des insaponifiables tel que les concentrats de tournesol, les concentrats d'avocat et les concentrats de soja, l'oxyde de zinc, les vitamines, les peptides et sucres végétaux, les anti-inflammatoires et les activateurs de la différenciation et/ou de la prolifération des kératinocytes.

9. Sucre en C7 choisi dans le groupe constitué par le mannoheptulose, les mélanges de mannoheptulose et de perséitol, pour son utilisation chez le bébé et l'enfant de bas âge dans la prévention des candidoses et des pityrosporoses choisies dans le groupe constitué par le pityriasis versicolor, la folliculite pityrosporique et la dermite séborrhéique en provoquant l'aggrégation des levures du genre candida et pityrosporum.

## Patentansprüche

1. Verwendung von mindestens einem C₇-Zucker, ausgewählt aus der Gruppe, die aus Mannoheptulose, den Mischungen von Mannoheptulose und von Perseitol gebildet wird, für die Herstellung einer Zusammensetzung, die für die Verhütung und/oder für die Behandlung von Mykosen, ausgewählt aus der Gruppe, die aus
- den Candidosen und
- den Pityrosporosen, ausgewählt aus der Gruppe, die aus Pityriasis versicolor, Pityrosporum-Folliculitis und seborrhoischer Dermatitis gebildet wird, insbesondere der seborrhoischen Dermatitis,
gebildet wird, beim Säugling und Kindern niedrigen Alters bestimmt ist, indem eine Aggregation der Hefen der Gattung Candida und Pityrosporum hervorgerufen wird.

2. Verwendung nach Anspruch 1, **dadurch gekennzeichnet, dass** die Candidosen aus der Gruppe, die aus den infantilen genitoglutäalen Candidosen, insbesondere dem glutäalen Erythem, gebildet wird, ausgewählt sind.

3. Verwendung nach einem der vorangegangenen Ansprüche, **dadurch gekennzeichnet, dass** die Quelle von C₇-Zuckern ein wasserlöslicher Extrakt von Zuckern von Avocado ist.

4. Verwendung nach einem der vorangegangenen Ansprüche, **dadurch gekennzeichnet, dass** die Quelle von C₇-Zuckern ein wasserlöslicher Extrakt von Zuckern von Avocado ist, der erhalten werden kann durch ein Verfahren, dass die folgenden, aufeinander folgenden Schritte umfasst:
Gewinnung eines Ölkuchens von Avocado, in vorteilhafter Weise von der Frucht der Avocado, durch Trocknen der Avocado, dann Extraktion der Lipide; dann
- kryogene Zerkleinerung, vollständige Delipidierung und wässrig-alkoholische oder wässrige Extraktion des Ölkuchens, dann Dekantation und Zentrifugation, um eine an C₇-Zuckern reiche, lösliche Fraktion zu gewinnen (Entfernung des Kuchens);
- Entmineralisierung der löslichen Fraktion, die in dem vorangegangenen Schritt erhalten worden ist, an einem ionischen Harz; dann
- Ultrafiltration bei 10000 Dalton; und
- Aufkonzentration unter Vakuum und Konditionierung.

5. Verwendung nach einem der vorangegangenen Ansprüche, **dadurch gekennzeichnet, dass** die Quelle von C₇-Zuckern ein wasserlöslicher Extrakt von Zuckern von Avocado ist, der bezogen auf das Gesamtgewicht der Trockensubstanz des Extrakts umfasst (relative Zusammensetzung durch HPLC bestimmt):
- D-Mannoheptulose 1 bis 90 Gew.-%
- Perseitol 1 bis 90 Gew.-%
- Saccharose unter 10 Gew.-%
- Glucose unter 10 Gew.-%
- Fructose unter 10 Gew.-%.

6. Verwendung nach einem der vorangegangenen Ansprüche, **dadurch gekennzeichnet, dass** die Zusammensetzung 0,001 bis 20 Gew.-% an C₇-Zuckern bezogen auf das Gesamtgewicht der Zusammensetzung umfasst.

7. Verwendung nach einem der Ansprüche 3 bis 6, **dadurch gekennzeichnet, dass** die Zusammensetzung 0,01 bis 20 Gew.-% des wasserlöslichen Extrakts von Zuckern von Avocado bezogen auf das Gesamtgewicht der Zusammensetzung umfasst.

8. Verwendung nach einem der vorangegangenen Ansprüche, **dadurch gekennzeichnet, dass** die Zusammensetzung außerdem mindestens einen Wirkstoff, ausgewählt aus der Gruppe, die aus den Antimykotika, den antibakteriellen Mitteln, den Antibiotika, den antiviralen Mitteln, den lipidischen pflanzlichen Extrakten, welche unverseifbare Anteile enthalten, wie die Sonnenblumen-Konzentrate, die Avocado-Konzentrate und die Soja-Konzentrate, Zinkoxid, den Vitaminen, den pflanzlichen Peptiden und Zuckern, den entzündungshemmenden Mitteln und den Aktivatoren der Differenzierung und/oder der Proliferation der Keratinozyten gebildet wird, umfasst.

9. C₇-Zucker, der aus der Gruppe, die aus Mannoheptulose, den Mischungen von Mannoheptulose und von Perseitol gebildet wird, ausgewählt ist, für dessen Verwendung bei der Verhütung von Candidosen und Pityrosporosen, ausgewählt aus der Gruppe, die aus Pityriasis versicolor, Pityrosporum-Folliculitis und seborrhoischer Dermatitis gebildet wird, beim Säugling und Kindern niedrigen Alters, indem eine Aggregation der Hefen der Gattung Candida und Pityrosporum hervorgerufen wird.

## Claims

1. Use of at least one C7 sugar selected from the group comprised of mannoheptulose and mixtures of mannoheptulose and perseitol for the manufacture a composition intended for the prevention and/or treatment, in babies and young infants, of mycoses selected from the group comprised of
- candidiases, and
- pityrosporoses selected from the group comprised of pityriasis versicolor, pityriosporum folliculitis and seborrheic dermatitis, in particular seborrheic dermatitis
by causing the aggregation of yeasts of the genus *Candida* and *Pityrosporum.*

2. The use according to claim 1, **characterized in that** the candidiases are selected from the group comprised of infantile candidiasis of the genitals and buttocks, in particular diaper rash.

3. The use according to any of the preceding claims, **characterized in that** the source of the C7 sugars is a water-soluble extract of avocado sugars.

4. The use according to any of the preceding claims, **characterized in that** the source of the C7 sugars is a water-soluble extract of avocado sugars capable of being obtained by a method comprising the following successive steps:
- obtaining an avocado oil cake, advantageously of the fruit of avocado, by drying the avocado and then extracting the fats; then
- cryogrinding, complete delipidation and hydroalcoholic or aqueous extraction of said oil cake, then decantation and centrifugation in order to recover a soluble fraction rich in C7 sugars (elimination of the cake);
- demineralization on ionic resin of said soluble fraction obtained in the preceding step; then
- ultrafiltration at 10,000 daltons; and
- vacuum concentration and conditioning.

5. The use according to any of the preceding claims, **characterized in that** the source of the C7 sugars is a water-soluble extract of avocado sugars which comprises by weight, compared to the total weight of dry matter of the extract (relative composition determined by HPLC):
- D-mannoheptulose 1% to 90%
- Perseitol 1% to 90%
- Sucrose less than 10%
- Glucose less than 10%
- Fructose less than 10%

6. The use according to any of the preceding claims, **characterized in that** the composition comprises 0.001% to 20% by weight of C7 sugar, compared to the total weight of the composition.

7. The use according to any of claims 3 to 6, **characterized in that** the composition comprises 0.01% to 20% by weight of said water-soluble extract of avocado sugars, compared to the total weight of the composition.

8. The use according to any of the preceding claims, **characterized in that** the composition further comprises at least one active ingredient selected from the group comprised of antifungals, antibacterials, antibiotics, antivirals, lipid vegetable extracts containing unsaponifiables such as sunflower concentrates, avocado concentrates and soy concentrates, zinc oxide, vitamins, plant peptides and sugars, anti-inflammatories and keratinocyte differentiation and/or proliferation activators.

9. C7 sugar selected from the group comprised of mannoheptulose and mixtures of mannoheptulose and perseitol for its use in babies and young infants in the prevention of candidiase and pityrosporoses selected from the group comprised of pityriasis versicolor, pityriosporum folliculitis and seborrheic dermatitis by causing the aggregation of yeasts of the genus *Candida* and *Pityrosporum.*
